# EUROPEAN PATENT APPLICATION

(11) **EP 3 000 485 A1**
(43) Date of publication of application: **30.03.2016**
(21) Application number: 14794213.0
(22) Date of filing: 06.05.2014
(51) Int. Cl.: A61L 9/12

(54) **CONCEALED AND INTELLIGENT DEVICE FOR IMPROVING THE ATMOSPHERE OR AIR INSIDE THE CAGES OF LIFTS, CAR LIFTS OR GOODS LIFTS, PURIFYING, DEODORISING AND HYGIENISING SAME**

(30) Priority: 09.05.2013 ES 201300474
(71) Applicant: Calderon Garcia, Jose Carlos, 30009 Murcia (ES)
(72) Inventor: Calderon Garcia, Jose Carlos, 30009 Murcia (ES)
(74) Representative: Capitán García, Maria Nuria
(86) International application number: PCT/ES2014/000069
(87) International publication number: WO 2014/181008

(57) **Abstract**

The invention relates to a concealed and intelligent device for improving the atmosphere or air inside a cage of any type of closed lift apparatus, which can combine the mixture of hygieniser and deodoriser products with the action of the ozone generator, in order to definitively hygienise, deodorise and improve the quality of the atmosphere and air inside a closed lift apparatus.

## Description

### OBJECT OF THE INVENTION

The invention relates to a device for improving the micro-atmosphere or environment inside a cage of any type of lift apparatus, thereby purifying, hygienising, deodorising and freshening the air contained therein.

### BACKGROUND OF THE INVENTION

As shown in patent publications ES 2 371 206 T3, ES 2 358 428 T3, ES 2 370 823 T3, the various existing devices for purifying, deodorising or treating the air of any space, consisting of a single body with a specific volume in which the inlet and outlet of the air to treat is placed.

Some treatment devices for improving air quality including deodorising same are merely parts integrated in a general air treatment system. Traditionally, existing mechanisms do not allow an intelligent control of the operation of the device, which is instead preprogrammed or continuous.

Existing air treatment devices including air purification and deodorising do not self-adapt to the needs appearing in the environment thereof, and thereby cannot fully optimise the operation system, electrical consumption and the resulting supply to the environment.

The existing devices do not provide a specific design that allows the installation thereof in a cage of any lift device.

The systems developed for air treatment do not consider the possibility of using an ozonising and pulverising system in an individual or combined manner.

To solve these drawbacks, the present patent application presents an adaptation of the design for installation on any lift device cage, the air outlet comprising a flexible duct that is easy to install, taking the treated air to the interior of the cage. In addition, a simple access is provided to the entire mechanism.

The device of this patent application comprises a fully autonomous and intelligent control or self-management system for the mechanism, obtaining data by a sensor incorporated in the chassis and subsequently conveying the data to a microchip that acts as a computer. Said microchip determines the appropriate work rate in view of the parameters received, thereby achieving the most economical and sustainable work mode possible. The issue of a simultaneous or independent execution of the air freshening function by ozonisation and pulverisation is solved, providing in the air purification process an air freshening system.

### DESCRIPTION OF THE INVENTION

The present invention is established and characterised in the independent claims, while the dependent claims describe additional characteristics thereof.

It is a device that is concealed from users of lift devices, all of the actuation and power supply mechanism being located on the outside of the lift device cage, meant to maintain ideal odour and hygiene conditions in the microatmosphere inside said closed space. Due to its versatile design it can be installed on both the vertical and horizontal outer wall. It has a chassis and a cover, both made of chlorine-free plastic, with a special guide design that provides easy access to maintenance technicians.

The mechanism or device is installed in the cages to prevent situations that can have negative consequences on the internal environment of the cage due to lack of personal hygiene of users, the appearance of bacteria in the lift pit with moisture and water, transporting rubbish or animals inside the same, or any element which when introduced in the cage can lead to a situation that will affect the internal environment thereof to a greater or lesser extent. This is the purpose of this system, which operates continuously and in a controlled manner over time, deodorising and hygienising the closed space used by the users. The device has two modes of operation that can be used simultaneously or separately. On one hand there is a small ozone generator, sufficiently tried and tested worldwide and in numerous applications, which constitutes the main air purification system. On the other hand there is a vaporisation system for various products, which can be complemented with hygienisers in order to hygienise the surfaces of the cage walls, as well as an air freshener for a full treatment of the cage, mixed with the treated air by an electrovalve controlled by the microprocessor. In addition, active charcoal and individual HEPA filters can be installed individually or jointly.

The only visible part of the mechanism or device inside the cage is a nozzle with an embellisher through which the treated air mixture is introduced from the air ducts, driven by the fan. This is an intelligent and configurable device that detects the activity inside the cage using a high-capacity sensor, self-programmed to optimise the operational mode and thereby save energy, product and be as sustainable as possible.

The device allows configuring several types of parameters for the self-configuration of the system, which will carry out its activity in an optimised manner according to the characteristics and dimensions of the closed space where it is installed, thereby preventing unnecessary costs. In addition it is powered independently of the lift device power supply, with an electrical assembly protection system.

The system has a system that alerts of the interior condition of the mechanism, such as malfunction, product levels and condition of the filters. This system uses LEDs in the low range and an LCD display in the high range. A study performed on the lift market in Spain has revealed the absence of a specific device for performing this function. Accordingly, there is a need in all cases, and particularly in lifts used to carry people, to install a system that acts in the lifts to provide improved and healthier conditions inside such a small space with limited ventilation.

The invention is applicable to all cage lift devices for persons, cars or goods. The installation of the invention is justified due to the unpleasant odour and lack of hygiene that may be present in lift pits, as well as those caused by other sources such as rubbish bags spilling their content, persons with poor hygiene, sick people potentially transmitting airborne diseases, etc.

### DESCRIPTION OF THE DRAWINGS

The present document is complemented with a set of drawings as an example for illustrating the preferred embodiment, and which in no way limits the invention.
Figure 1 shows a drawing of the entire system.
Figure 2 shows a drawing of the body of the system, with the cover displaced vertically upwards.

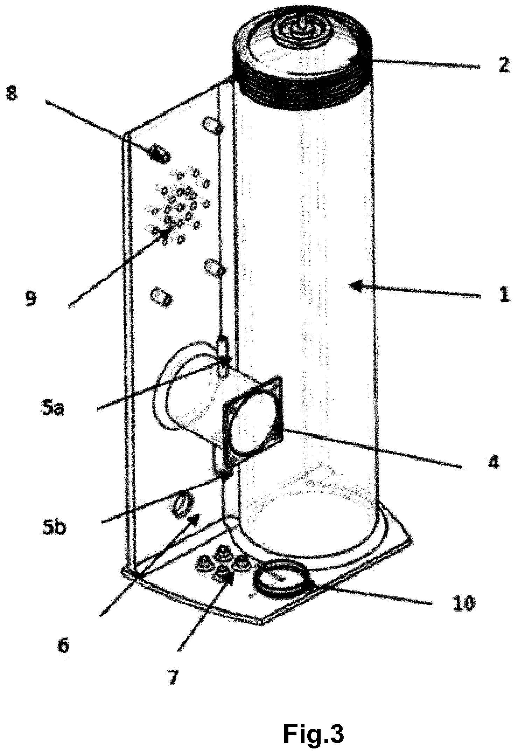

Figure 3 shows a drawing of the internal part of the device.
Figure 4 shows a drawing of the visible part and the elements for attachment to the cage on which it is installed.
Figure 5 shows a cross-section of the elements shown in Figure 4.

### PREFERRED EMBODIMENT OF THE INVENTION

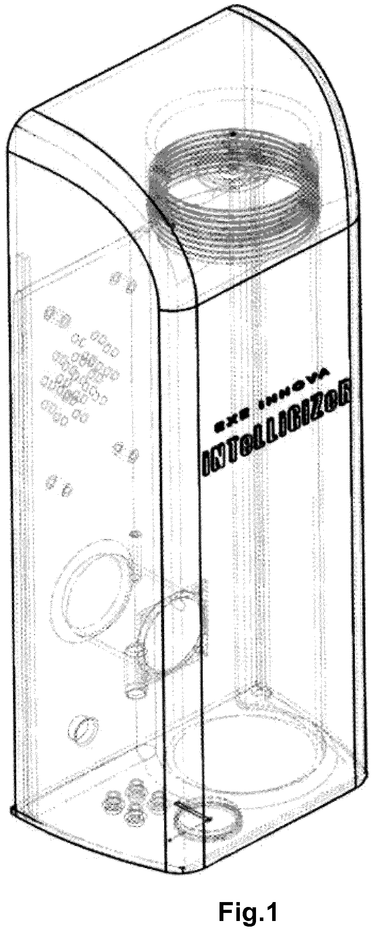

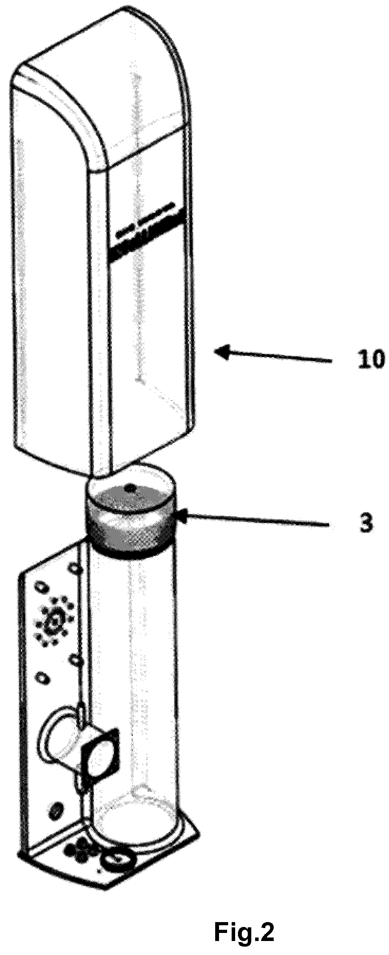

The concealed and intelligent device for deodorising and hygienising the environment or microatmosphere inside a lift device comprises a plastic body (figure 1) with a hollow cylinder (1) filled with an aerosol (2) containing the hygienising and/or deodorising product, sealed by a securing element (3) shown in figure 2.

The hygienising and deodorising product is injected through the inlet tube (5a) into a recombination chamber where the air outlet from the ozone generator (4) is located, where it is mixed with air.

This mixture is distributed inside the closed space by a fan connected to the recombination chamber which takes the air flow from the ozone generator and leads it to a duct which guides the mixture from the outlet tube (5b) to the interior through the orifice (6), through a duct similar to the silicone tubes, which are particularly appropriate for the system.

The flow of hygienising and deodorising product and the activation of the ozone generator is controlled by a flow control element as that placed in (7). This flow controller is governed by an electronic system secured to (8) by attachment elements on a vent (9). The system analyses the surroundings with a sensor housed in (10), detecting the presence or activity inside the closed space, thereby optimising the activity of the system. The system gathers information on the environment and switches to standby mode after being inactive for a specified time, thereby preventing an unnecessary use of deodorising and/or hygienising product and reducing power consumption.

The system is assembled as shown in figure 2, with a cover (10) provided with guides used to introduce the body.

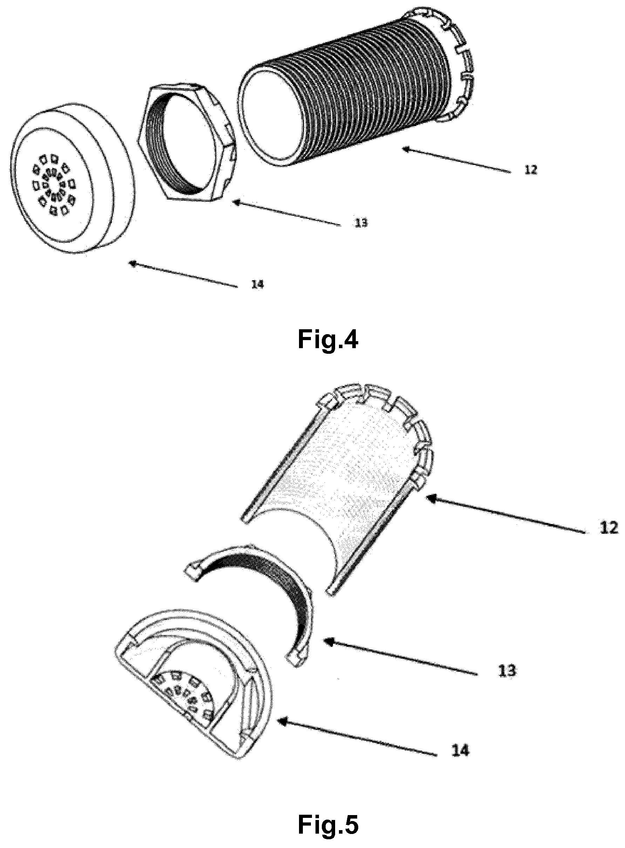

In addition, the part installed in the closed space, that is, the part that is visible, and the attachments thereof consists of three parts shown in figure 4 and figure 5, consisting of a threaded plastic lug (12) by way of a cable gland with a length from 5 to 30 cm. and a diameter from 1 to 10 cm. An attachment element (13) made of a plastic material that screws inside the aforementioned part (12) is provided with a ribs for the pressure attachment of the embellisher (14), which consists of a cylindrical plastic part characterised by spaces, an inner stop to prevent rotation of said part, and a cylindrical wall in the form of a cylindrical connector where the installed duct is connected, through which the mixture of ozone-purified air and deodorising and hygienising product is guided.

The operational system of the mechanism can be independent, selecting between using the ozone generator only without applying hygienising and/or deodorising product, or inversely applying only hygienising and/or deodorising product without ozone, or both systems can operate simultaneously for maximum effectiveness.

The casing includes an alert system for indicating the level of the supply products, either hygieniser or air freshener.

## Claims

1. Concealed and intelligent device for improving the atmosphere or air inside the cages of lifts, car lifts or goods lifts, purifying, deodorising and hygienising same, **characterised in that** it consists of the following elements:
- a chassis and cover of a lightweight, chlorine-free material,
- an electrical protection system,
- a fully hermetic aerosol deposit,
- an ozone generator,
- an impeller fan,
- an electronic element in which the microprocessor is housed,
- a transmitting sensor,
- a gas electrovalve,
- air ducts.

2. Concealed and intelligent device for improving the atmosphere or air inside the cages of lifts, car lifts or goods lifts, purifying, deodorising and hygienising same according to claim 1, **characterised by** comprising an intelligent application system consisting of a transmitting sensor and an electronic board with the microcontroller which adapts the operation of the system in an autonomous manner to the use of each lift device.

3. Concealed and intelligent device for improving the atmosphere or air inside the cages of lifts, car lifts or goods lifts, purifying, deodorising and hygienising same according to the preceding claims, **characterised by** combining in a single mechanism an ozone hygienisation system and an indirect-spray air freshener, where the two can be combined or only the spray system used, adding a hygienising product to the spray, the two systems operating either simultaneously or each one independently.

4. Concealed and intelligent device for improving the atmosphere or air inside the cages of lifts, car lifts or goods lifts, purifying, deodorising and hygienising same according to the preceding claims, **characterised in that** it is designed specifically to be installed in cages of lift devices such as lifts, car lifts, good lifts and other, designed with small dimensions.

5. Concealed and intelligent device for improving the atmosphere or air inside the cages of lifts, car lifts or goods lifts, purifying, deodorising and hygienising same according to the preceding claims, **characterised in that** the atmosphere inside the cage is treated and renewed by injecting purified air, optionally reinforcing the hygienisation and air freshening action by applying hygieniser and/or air freshener in spray form.

6. Concealed and intelligent device for improving the atmosphere or air inside the cages of lifts, car lifts or goods lifts, purifying, deodorising and hygienising same according to the preceding claims, **characterised in that** the condition of the system is indicated by a group of LEDs of various colours in the basic range, or an LCD display in the high range.

7. Concealed and intelligent device for improving the atmosphere or air inside the cages of lifts, car lifts or goods lifts, purifying, deodorising and hygienising same according to the preceding claims, **characterised by** having a structure that is adaptable to both vertical walls with screwed brackets, further provided with two attachment tabs, and to horizontal walls using attachment brackets.

8. Concealed and intelligent device for improving the atmosphere or air inside the cages of lifts, car lifts or goods lifts, purifying, deodorising and hygienising same according to the preceding claims, **characterised by** having an external power supply that does not depend on the lift device mechanism.

9. Concealed and intelligent device for improving the atmosphere or air inside the cages of lifts, car lifts or goods lifts, purifying, deodorising and hygienising same according to the preceding claims, **characterised in that** the purified air supply nozzle is inside the cage, assembled by a threaded system.

10. Concealed and intelligent device for improving the atmosphere or air inside the cages of lifts, car lifts or goods lifts, purifying, deodorising and hygienising same according to the preceding claims, **characterised by** having a filter for cleaning the air treated inside the mechanism, a HEPA filter and an active charcoal filter.
